# EUROPEAN PATENT APPLICATION

(11) **EP 3 181 037 A1**
(43) Date of publication of application: **21.06.2017**
(21) Application number: 16204023.2
(22) Date of filing: 14.12.2016
(51) Int. Cl.: A61B 5/00, A61B 5/1486, A61B 5/145, A61B 5/15, A61B 5/157

(54) **ELECTRODE NEEDLE, BIOLOGICAL INFORMATION MEASURING DEVICE, AND LIQUID SUPPLY DEVICE**

(30) Priority: 14.12.2015 JP 2015242928
(71) Applicant: Seiko Epson Corporation, Tokyo 160-8801 (JP)
(72) Inventor: Fujita, Tetsuji, Nagano, 392-8502 (JP); Ito, Masaki, Nagano, 392-8502 (JP)
(74) Representative: Hoffmann Eitle

(57) **Abstract**

An electrode needle (a first electrode needle) is an electrode needle punctured into an organism. The electrode needle includes a columnar or tubular core section, an electrode formed in the core section, an insulating section in which the electrode is covered with an insulating material, and an electrode window section in which the electrode or a sensing layer (a detection layer) formed on the electrode is exposed.

## Description

### BACKGROUND

### 1. Technical Field

The present invention relates to an electrode needle, a biological information measuring device, and a liquid supply device.

### 2. Related Art

A diabetic having abnormal insulin secretion from the pancreas, in particular, no insulin secretion performs blood sampling, for example, four times in one day before meals and before bedtime and measures blood sugar levels. When a blood sugar level is high, the diabetic performs insulin administration.

JP-T-2010-537732 (Patent Literature 1) discloses a continuous glucose monitoring device that continuously and automatically measures blood sugar levels of a diabetic. The continuous glucose monitoring device is called CGM. In the CGM, enzyme reaction is used for measurement of glucose.

JP-T-2005-520648 (Patent Literature 2) discloses an insertion needle for inserting a subcutaneous insertion-type device. The insertion needle includes a rectangular needle main body and a distal end portion. The distal end portion has a substantially smooth surface inclining toward a nib and a distal end to enable insertion of a needle into under the skin. The needle main body includes, along a part thereof, an elongated groove in which at least a part of the subcutaneous insertion-type device can be housed.

In Patent Literature 1, a cylindrical needle is used. A substrate is set in the needle. A plurality of electrodes are set on the substrate. After the needle is inserted into a skin tissue of a patient, the needle is pulled out leaving the substrate in the skin tissue. Since the substrate is set in the needle in this way, the external shape of the needle is large. Therefore, when the needle is inserted into the skin tissue, the needle easily gives pain to the patient. In Patent Literature 2, the insertion needle includes the smooth surface inclining toward the distal end and the elongated grove and inserts the subcutaneous insertion-type device into a skin tissue. However, the external shape of the insertion needle is large. Therefore, when the subcutaneous insertion-type device is inserted into the skin tissue, the subcutaneous insertion-type device easily gives pain to the patient.

Therefore, there is a demand for an electrode needle that highly accurately measures information in an organism and prevents a patient from easily feeling pain when the needle is inserted and when the needle is pulled out, a biological information measuring device including the electrode needle, and a liquid supply device.

### SUMMARY

An advantage of some aspects of the invention is to solve at least a part of the problems, and the invention can be implemented as the following forms or application examples.

### Application Example 1

An electrode needle according to this application example is an electrode needle punctured into an organism, and including: a columnar or tubular core section; an electrode formed in the core section; an insulating section in which the electrode is covered with an insulating material; and an electrode window section in which the electrode or a sensing layer formed on the electrode is exposed.

The electrode needle includes the core section, the electrode formed in the core section, the insulating section in which the electrode is covered with the insulating material, and the electrode window section in which the electrode or the sensing layer is exposed. Consequently, since a region other than the electrode window section is covered with the insulating section, when the electrode needle is punctured into the organism and used, it is possible reduce a loss of a voltage, noise, and the like and accurately detect, for example, an electric current. Consequently, it is possible to realize the electrode needle that can highly accurately measure information in the organism.

### Application Example 2

In the electrode needle according to the application example, it is preferable that the electrode window section is exposed in a rectangular shape along a puncturing direction in which the electrode needle is punctured.

With this electrode needle, since the electrode window section is exposed in the rectangular shape along the puncturing direction in which the electrode needle is punctured, it is possible to prevent peeling of the electrode, the sensing layer, and the like during the puncture.

### Application Example 3

In the electrode needle according to the application example, it is preferable that the core section or the insulating section includes inclined surface sections inclining toward the electrode window section.

With this electrode needle, since the core section or the insulating section includes the inclined surface sections inclining toward the electrode window section, it is possible to reduce resistance at the time when the electrode needle is inserted and pulled out. Therefore, it is possible to realize the electrode needle that prevents a patient from easily feeling pain.

### Application Example 4

In the electrode needle according to the application example, it is preferable that the sensing layer senses information in the organism.

With this electrode needle, the sensing layer can sense a predetermined component of the organism and feed an electric current.

### Application Example 5

In the electrode needle according to the application example, it is preferable that a maj or diameter of the electrode needle is 0.1 mm or more and 0.5 mm or less.

With this electrode needle, the major diameter of the electrode needle is 0.1 mm or more. Note that the major diameter indicates a diameter in a place where the diameter is the largest in a sectional shape of the electrode needle. In this case, since the electrode needle sticks into the skin without bending, it is possible to prevent the electrode needle from being easily broken. The major diameter of the electrode needle is 0.5 mm or less. In this case, since a stimulus of the electrode needle to a pain spot is small, it is possible to prevent a patient from easily feeling pain. Therefore, it is possible to realize the electrode needle that is less easily broken and prevents the patient from easily feeling pain.

### Application Example 6

In the electrode needle according to the application example, it is preferable that angles formed by the electrode window section and the inclined surface sections are 90° or more and 170° or less.

With this electrode needle, the angles formed by the electrode window section and the inclined surface sections are 90° or more and 170° or less. Therefore, it is possible to reduce resistance at the time when insertion and pull-out of the electrode needle is performed and smoothly perform the insertion and the pull-out.

### Application Example 7

In the electrode needle according to the application example, it is preferable that the electrode needle includes a channel for circulating liquid.

With this electrode needle, since the channel for circulating the liquid is provided, it is possible to not only measure the information in the organism but also include a function of an injection needle.

### Application Example 8

A biological information measuring device according to this application example includes: a first electrode needle and a second electrode needle configured by the electrode needle according to any of the application examples; and a current detecting section configured to detect an electric current between the first electrode needle and the second electrode needle.

The biological information measuring device includes the first electrode needle and the second electrode needle. By detecting, with the current detecting section, a value of the electric current between the first electrode needle and the second electrode needle, it is possible to measure biological information. Note that the first electrode needle and the second electrode needle are respectively inserted into different parts. Therefore, compared with when a needle including a substrate on which the first electrode and the second electrode are set as in the configuration in the past is used, it is possible to reduce a major diameter of the first electrode needle and the second electrode needle inserted into the skin. Note that, as the major diameter is smaller, a pain spot of the skin is less easily stimulated. Therefore, in the biological information measuring device in this application example, even if the first electrode needle and the second electrode needle are inserted into or pulled out from the abdomen or the like of a patient in order to measure information in the organism, it is possible to prevent the patient from easily feeling pain.

### Application Example 9

In the biological information measuring device according to the application example, it is preferable that the information in the organism is information concerning glucose, the first electrode needle is a working electrode including a sensing layer including an enzyme layer, and the second electrode is a counter electrode that leads an electric current between the second electrode needle and the first electrode needle.

With this biological information measuring device, since the first electrode needle is the working electrode including the sensing layer including the enzyme layer, the glucose reacts with the enzyme layer and generates an electric current. The second electrode needle is the counter electrode that leads an electric current between the second electrode needle and the first electrode needle. Note that, as glucose concentration is higher, the current value is larger because the reaction with the enzyme layer further advances. Therefore, it is possible to accurately detect the concentration of the glucose by analyzing the current value detected by the current detecting section.

### Application Example 10

In the biological information measuring device according to the application example, it is preferable that the biological information measuring device includes a third electrode needle functioning as a reference electrode used for detection of resistance of an electric current flowing to the first electrode needle.

The biological information measuring device includes the third electrode needle (the reference electrode) used for detection of resistance of an electric current flowing to the first electrode needle. Interstitial fluid is present in the organism. When a substance included in the interstitial fluid adheres to the surfaces of the first electrode needle and the second electrode needle and films are formed, resistance applied to an electric current flowing to the first electrode needle and the second electrode needle increases. Therefore, by detecting the resistance using the reference electrode, it is possible to improve measurement accuracy of the current value in the current detecting section.

### Application Example 11

A liquid supply device according to this application example includes the biological information measuring device according to the application examples described above. The first electrode needle or the second electrode needle includes a channel for circulating liquid.

The liquid supply device includes the biological information measuring device (the first electrode needle (the working electrode) and the second electrode needle (the counter electrode)). Therefore, it is possible to detect an electric current and measure information in an organism. Since the first electrode needle or the second electrode needle includes the channel for circulating liquid, the liquid supply device does not need to separately include an injection needle. It is possible to discharge the liquid from the channel. Therefore, it is possible to realize the liquid supply device that prevents a patient from easily feeling pain. Further, it is possible to achieve simplification of the liquid supply device. It is possible to improve convenience.

### Application Example 12

A liquid supply device according to this application example includes the biological information measuring device according to the application examples described above. Any one of the first electrode needle, the second electrode needle, and the third electrode needle includes a channel for circulating liquid.

The liquid supply device includes the biological information measuring device (the first electrode needle (the working electrode), the second electrode needle (the counter electrode), and the third electrode needle (the reference electrode)). Therefore, it is possible to detect an electric current and measure information in an organism. Since any one of the first electrode needle, the second electrode needle, and the third electrode needle includes the channel for circulating liquid, the liquid supply device does not need to separately include an injection needle. It is possible to discharge the liquid from the channel. Therefore, it is possible to realize the liquid supply device that prevents a patient from easily feeling pain. Further, it is possible to achieve simplification of the liquid supply device. It is possible to improve convenience.

### Application Example 13

In the liquid supply device according to the application example, it is preferable that the information in the organism is information concerning glucose, and the liquid is insulin.

The liquid supply device detects the glucose in the organism and supplies the insulin into the organism. Therefore, the liquid supply device can supply the insulin according to an amount of the glucose in the organism.

### BRIEF DESCRIPTION OF THE DRAWINGS

The invention will be described with reference to the accompanying drawings, wherein like numbers reference like elements.
Fig. 1 is a schematic perspective view of a measuring device according to a first embodiment.
Fig. 2 is a schematic perspective view of the measuring device.
Fig. 3 is a schematic side sectional view for explaining a first electrode needle to a third electrode needle.
Fig. 4 is a general side view schematically showing the first electrode needle.
Fig. 5A is a sectional view schematically showing the cross section of the first electrode needle.
Fig. 5B is a sectional view schematically showing the cross section of the second electrode needle and the third electrode needle.
Fig. 6A is a general side view schematically showing a manufacturing method (a preparing step) for an electrode needle.
Fig. 6B is a general side view schematically showing the manufacturing method (a polishing step) for the electrode needle.
Fig. 6C is a general side view schematically showing the manufacturing method (a cutting step) for the electrode needle.
Fig. 6D is a general side view schematically showing the manufacturing method (a first insulating film forming step) for the electrode needle.
Fig. 6E is a general side view schematically showing the manufacturing method (a wiring step) for the electrode needle.
Fig. 6F is a general side view schematically showing the manufacturing method (a second insulating film forming step) for the electrode needle.
Fig. 6G is a general side view schematically showing the manufacturing method (a sensor forming step) for the electrode needle.
Fig. 7 is a general sectional view schematically showing the structure of a detection film formed on a surface section of the first electrode needle.
Fig. 8 is a block diagram schematically showing a circuit configuration of the measuring device.
Fig. 9 is a circuit diagram showing the configuration of a current detection circuit.
Fig. 10 is a general perspective view schematically showing another example of a surface section according to a second embodiment.
Fig. 11 is a schematic perspective view of a measuring device according to a third embodiment.
Fig. 12 is a circuit diagram showing the configuration of a current detection circuit.
Fig. 13 is a general perspective view schematically showing the structure of an insulin supply device according to a fourth embodiment.
Fig. 14 is a general side view schematically showing the structure of a first electrode needle.
Fig. 15 is a main part side view schematically showing the structure of the first electrode needle.
Fig. 16 is a block diagram schematically showing a circuit configuration of the insulin supply device.
Fig. 17 is a block diagram schematically showing a circuit configuration of an insulin supply device according to a fifth embodiment.
Fig. 18 is a block diagram schematically showing a circuit configuration of an insulin supply device according to a sixth embodiment.
Fig. 19 is a schematic perspective view showing the structure of an insulin supply device according to a seventh embodiment.
Fig. 20 is a side sectional view schematically showing the structure of a first electrode needle to a third electrode needle.

### DESCRIPTION OF EXEMPLARY EMBODIMENTS

Embodiments are explained below with reference to the drawings.

Note that members in the drawings are shown with scales varied for each of the members in order to show the members in recognizable sizes on the drawings.

### First Embodiment

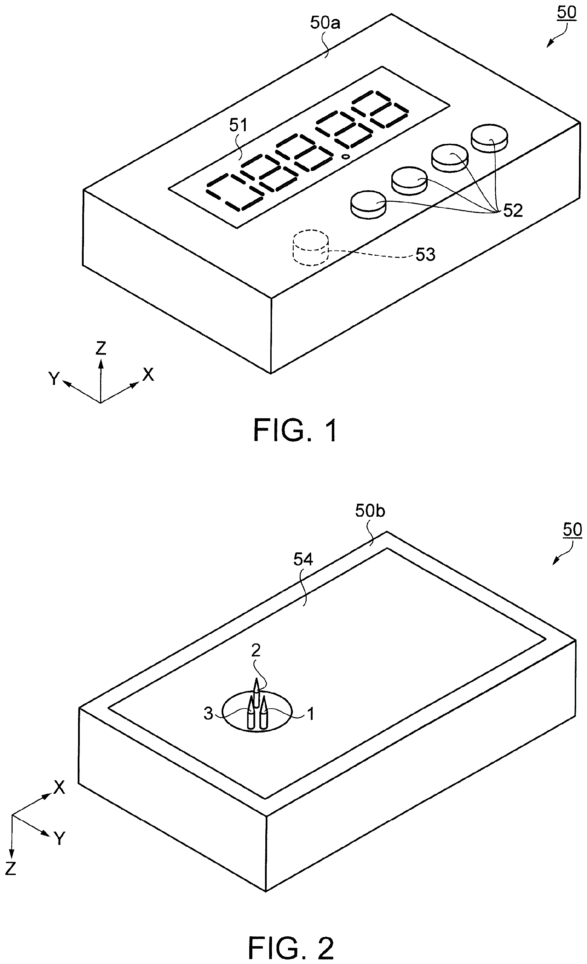

In a first embodiment, a characteristic example of a measuring device 50 functioning as a biological information measuring device that measures a glucose value is explained with reference to the drawings. Figs. 1 and 2 are schematic perspective views of the measuring device 50 according to the first embodiment. Specifically, Fig. 1 is a perspective view of the measuring device 50 viewed from the front side. Fig. 2 is a perspective view of the measuring device 50 viewed from the rear side.

As shown in Fig. 1, the measuring device 50 has a rectangular tabular shape as a plane shape. The longitudinal direction of the measuring device 50 is represented as an X direction and the thickness direction of the measuring device 50 is represented as a Z direction. A direction orthogonal to the X direction and the Z direction is represented as a Y direction. A surface on a +Z-direction side of the measuring device 50 is represented as a front surface 50a. A display section 51 and input sections 52 are set on the front surface 50a. The display section 51 displays a measured glucose value. As the display section 51, a display device such as an LCD (Liquid Crystal Display) or an OLED (Organic light-emitting diode) is used.

The input sections 52 are configured from push switches, rotary knobs, and the like. An operator operates the input sections 52 to perform instruction inputs of a measurement start instruction, a measurement interval, and the like for the measuring device 50. Further, the measuring device 50 includes a speaker 53. The speaker 53 warns when the measured glucose value is higher than a determination value.

As shown in Fig. 2, a surface on a -Z-direction side of the measuring device 50 is represented as a rear surface 50b. An adhesive sheet 54 is set on the rear surface 50b. The adhesive sheet 54 is used when a main body of the measuring device 50 is set and fixed on a skin of a subject. The main body of the measuring device 50 is stuck to the skin of the subject by the adhesive sheet 54. On the rear surface 50b, a first electrode needle 1 functioning as a working electrode, a second electrode needle 2 functioning as a counter electrode, and a third electrode needle 3 functioning as a reference electrode are set in a -X-direction side.

The first electrode needle 1 to the third electrode needle 3 are punctured into the skin of the subject and used. The first electrode needle 1 to the third electrode needle 3 are disposed in the positions of vertex angles of an isosceles triangle. The distance between the first electrode needle 1 and the third electrode needle 3 is shorter than the distance between the first electrode needle 1 and the second electrode needle 2 and is shorter than the distance between the second electrode needle 2 and the third electrode needle 3.

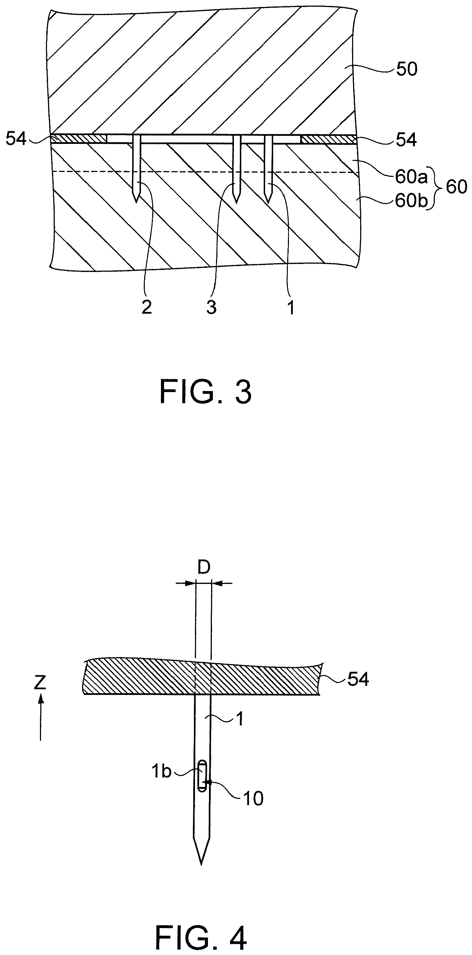

Fig. 3 is a schematic side sectional view for explaining the first electrode needle 1 to the third electrode needle 3.

As shown in Fig. 3, on the surface of a subject 60 serving as an organism, a corium (a skin tissue 60a) is disposed under a skin. A subcutaneous tissue 60b is disposed under the skin tissue 60a. The thickness of the skin tissue 60a is approximately 2 mm. The length of the first electrode needle 1 to the third electrode needle 3 is approximately 5 mm. In this embodiment, the first electrode needle 1 to the third electrode needle 3 have a columnar shape circular in section. The distal ends of the first electrode needle 1 to the third electrode needle 3 are pointed in a conical shape. The first electrode needle 1 to the third electrode needle 3 piece through parts of a skin surface 60c respectively corresponding thereto to be inserted (punctured) into the subject 60. The first electrode needle 1 to the third electrode needle 3 penetrate through the skin tissue 60a and reaches the subcutaneous tissue 60b. Note that interstitial fluid is filled in the subcutaneous tissue 60b.

A detection film 10 is set on the surface of the first electrode needle 1. The detection film 10 functions as a working electrode that reacts with the interstitial fluid. The second electrode needle 2 functions as a counter electrode. The third electrode needle 3 functions as a reference electrode. The first electrode needle 1 to the third electrode needle 3 configure an electrode that detects an electric current flowing in the interstitial fluid. The first electrode needle 1 and the second electrode needle 2 detect an electric current generated by the reaction. When films of protein or the like are formed on the surfaces of the electrode needles, the films cause electric resistance. The third electrode needle 3 is an electrode for detecting an increase in the electric resistance due to the films.

Fig. 4 is a general side view schematically showing the first electrode needle 1.

As shown in Fig. 4, the first electrode needle 1 has a columnar shape circular in section and is pointed at a distal end portion. In the first electrode needle 1, a surface section 1b explained below is formed from the center toward the distal end side. The detection film 10 is set on the surface section 1b.

A major diameter D of the first electrode needle 1 is 0.1 mm or more. The major diameter indicates a diameter of a place where the diameter is the largest in the sectional shape of the first electrode needle 1. In this case, the first electrode needle 1 can be prevented from easily broken. The major diameter D of the first electrode needle 1 is 0.5 mm or less. In this case, since a stimulus of the first electrode needle 1 to a pain spot is small, it is possible to prevent the subject 60 from easily feeling pain. Therefore, the first electrode needle 1 of the measuring device 50 can be formed as an electrode that is less easily broken and prevents the subject 60 from easily feeling pain. Further, the major diameter D of the first electrode needle 1 is desirably 0.15 mm or more and 0.25 mm or less. The first electrode needle 1 can be formed as an electrode that is much less easily broken and prevents the subject 60 from more easily feeling pain. Note that the major diameters of the second electrode needle 2 and the third electrode needle 3 are set the same as the major diameter D of the first electrode needle 1.

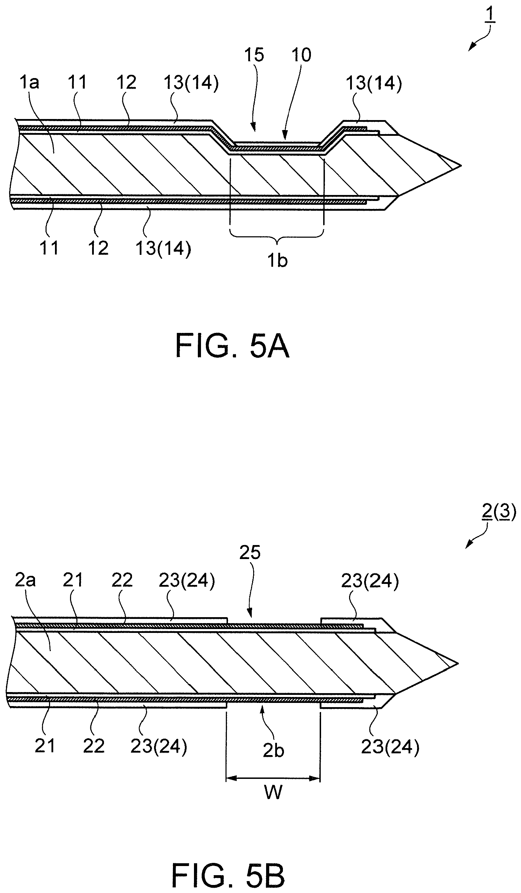

Fig. 5A is a sectional view schematically showing the cross section of the first electrode needle 1. Fig. 5B is a sectional view schematically showing the cross section of the second electrode needle 2 and the third electrode needle 3. Note that Fig. 5B shows the second electrode needle 2 as an example.

As the material of a core section 1a of the first electrode needle 1, in this embodiment, stainless steel (SUS), which is metal having a circular shape in section, is used. As shown in Fig. 5A, in the core section 1a functioning as a base material of an electrode needle formed in a columnar shape, the surface section 1b obtained by shaving a part of a side surface is formed. The detection film 10 is set on the surface section 1b. Note that, since the core section 1a is made of stainless steel having electric conductivity, a first insulating film 11 is formed on the side surface. An electrode layer 12 is formed on the outer surface of the first insulating film 11. A second insulating film 13 covered with an insulating material is formed on side surfaces other than the surface section 1b on the outer surface of the electrode layer 12. Note that the second insulating film 13 corresponds to an insulating section 14 in which an electrode (the electrode layer 12) is covered with the insulating material. Consequently, in the region of the surface section 1b, an electrode window section 15 in which the electrode (the electrode layer 12) is exposed to the outside is formed. Note that, as the electrode layer 12, in this embodiment, platinum (Pt) plating is applied. However, gold (Au) plating may be applied. Note that the detection film 10 is formed on the electrode window section 15. Details concerning the detection film 10 are explained below.

Like the first electrode needle 1, the material of a core section 2a of the second electrode needle 2 is stainless steel. As shown in Fig. 5B, like the first insulating film 11, a first insulating film 21 is formed on the core section 2a. Thereafter, an electrode layer 22 is formed on the outer surface of the first insulating film 21. A region 2b having predetermined width W around the core section 2a is exposed and a region other than the region 2b is covered with the insulating material, whereby a second insulating film 23 is formed. Note that the second insulating film 23 corresponds to an insulating section 24 in which an electrode (the electrode layer 22) is covered with the insulating material. Consequently, an electrode window section 25 in which the electrode (the electrode layer 22) is exposed to the outside is formed in the region 2b. Note that, as the electrode layer 22, in this embodiment, platinum (Pt) plating is applied. However, gold (Au) plating may be applied. The platinum has high stability and less easily reacts with the interstitial fluid and oxygen to be deteriorated. Therefore, the second electrode needle 2 can stably function for a long period.

The third electrode needle 3 is configured the same as the second electrode needle 2. As an electrode layer of the third electrode needle 3, in this embodiment, platinum (Pt) plating is applied. However, the electrode layer may be an electrode layer obtained by stacking silver (Ag) film on platinum. Note that, when the silver (Ag) film is tacked on the platinum, the silver reversibly transitions to forms of silver and a silver ion in the interstitial fluid. Therefore, when this reaction is used, the silver is suitable for detection of film formation on the surface of the third electrode needle 3. The third electrode needle 3 functions as a reference electrode for detecting glucose using an enzyme. The silver surface is more desirably converted into AgCl.

As the material of the core sections of the first electrode needle 1 to the third electrode needle 3, the stainless steel is used in this embodiment. However, metal other than the stainless steel, hard resin, an alloy, ceramics, or the like may be used. Note that, if the material is the metal or the alloy, the major diameters can be set small compared with when the material is the hard resin, the ceramics, or the like.

The measuring device 50 continuously performs detection of glucose in the interstitial fluid. The measuring device 50 can be used in a CGMS (Continuous Glucose Monitoring System) that continuously observes a glucose value in the interstitial fluid.

A detection method for glucose in the detection film 10 is explained. The detection film 10 includes an enzyme such as glucose oxidase. In the glucose, enzyme reaction indicated by Formula (1) is caused by the enzyme.

Glucose + O₂ + H₂O → gluconic acid + H₂O₂ (1)

Hydrogen peroxide is generated by the enzyme reaction. A voltage is applied to the hydrogen peroxide to electrolyze the hydrogen peroxide. The voltage is not particularly limited and only has to be determined by an experiment. In this embodiment, for example, a voltage of 0.6 V is applied.

Reaction indicated by Formula (2) is caused in the first electrode needle 1 by the electrolysis. Reaction indicated by Formula (3) is caused in the second electrode needle 2 by the electrolysis.

H₂O₂ → O₂ + H₂⁺ + 2e⁻ (2)

2H⁺ + 1/2O₂ + 2e⁻ → H₂O (3)

As indicated by Formula (2), oxygen, a hydrogen ion, and an electron are generated in the first electrode needle 1. As indicated by Formula (3), in the second electrode needle 2, the electron, the oxygen, and the hydrogen ion supplied from the first electrode needle 1 react, whereby hydroxide ion concentration increases. In this way, since the electron moves between the first electrode needle 1 and the second electrode needle 2, it is possible to quantize an amount of the hydrogen peroxide by measuring a current value. An amount of the hydrogen peroxide to be generated is detected to calculate an amount of the glucose.

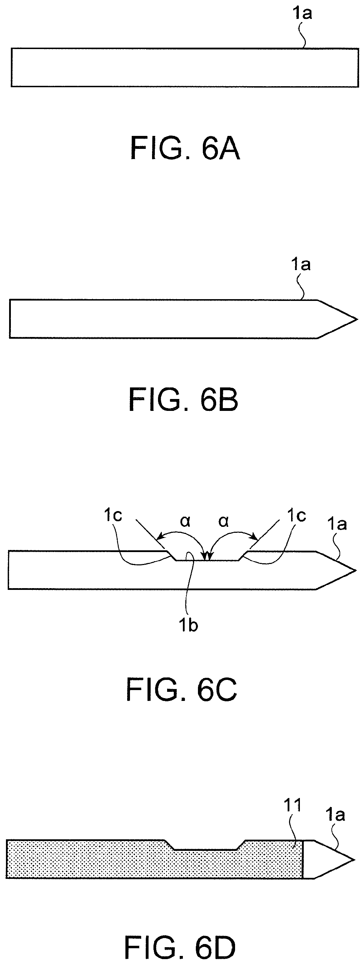

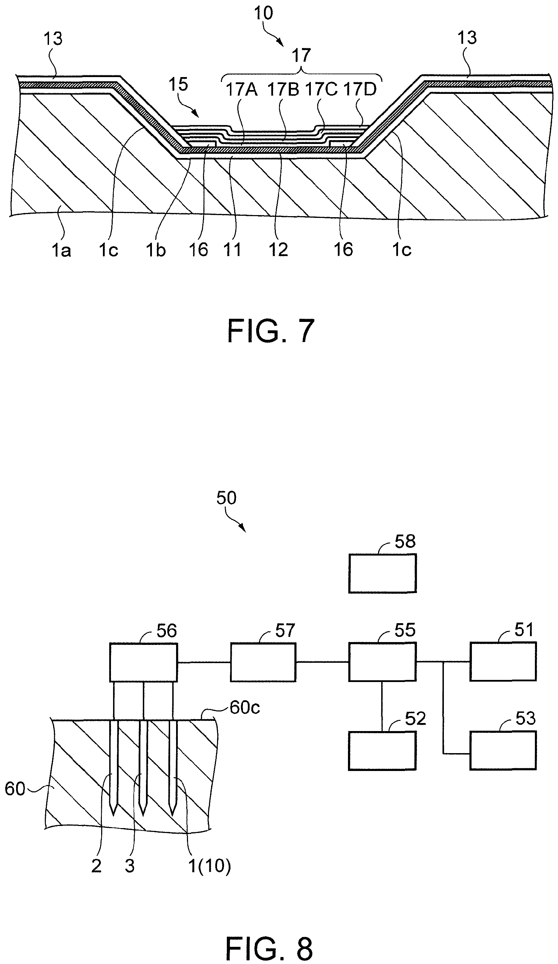

Figs. 6A to 6G are general side views schematically showing a manufacturing method for an electrode needle. Fig. 7 is a general sectional view schematically showing the structure of the detection film 10 formed on the surface section 1b of the first electrode needle 1. The manufacturing method for an electrode needle is explained with reference to the first electrode needle 1 as an example.

In a preparing step shown in Fig. 6A, a stainless steel bar to be formed as the core section 1a is prepared. Subsequently, in a polishing step shown in Fig. 6B, the distal end portion of the stainless steel bar is polished into a conical shape. The distal end portion is pointed at an acute angle. Subsequently, in a cutting step shown in Fig. 6C, the side surface of the core section 1a is shaved to form the surface section 1b to be formed as a flat surface. Note that, in this case, for example, a cutting blade is rotated to be orthogonal to the longitudinal direction of the core section 1a (a puncturing direction in which the electrode needle is punctured) to cut the core section 1a. Consequently, the surface section 1b is formed in a rectangular shape.

In the cutting step, the surface section 1b is cut to be tapered to form inclined surface sections 1c respectively expanding to the outer side in regions of steps extending from end portions (both end portions) in the longitudinal direction of the surface section 1b to side surfaces on both sides of the surface section 1b. Both of angles α formed by the surface section 1b and the inclined surface sections 1c are set to approximately 135° in this embodiment. Note that the angles α may be set as appropriate between 90° or more and 170° or less.

Note that, to put it differently, the core section 1a includes the inclined surface sections 1c inclining toward the electrode window section 15 explained below. The angles formed by the electrode window section 15 and the inclined surface sections 1c are 90° or more and 170° or less.

Since the core section 1a includes the inclined surface sections 1c, compared with when the inclined surface sections 1c are formed at less than 90°, it is possible to reduce resistance when the first electrode needle 1 is punctured into the skin surface 60c. Therefore, it is possible to prevent the subject 60 from easily feeling pain.

Subsequently, in a first insulting film forming step shown in Fig. 6D, the first insulating film 11 is formed on the side surface (including the surface section 1b and the inclined surface sections 1c) of the core section 1a excluding a needle tip. The first insulating film 11 is a film that insulates the core section 1a and the electrode layer 12 explained below. In this embodiment, synthetic resin is used as the insulating material. However, the insulating material may be a ceramics material. Note that, as the synthetic resin, thermoplastic resin such as polyamide (PA), polybutylene terephthalate (PBT), polycarbonate (PC), polypropylene (PP), polyamide-imide (PAI), polyimide (PI), polyethylene terephthalate (PET), and polyvinyl chloride (PVC) and thermosetting resin such as epoxy resin (EP), polyurethane (PUR), and unsaturated polyester (UP) can be used. An insulating film functioning as the first insulating film 11 may be formed by subjecting stainless steel to alumina treatment. The insulating film functioning as the first insulating film 11 may be formed by performing heat treatment using special stainless steel (KCF).

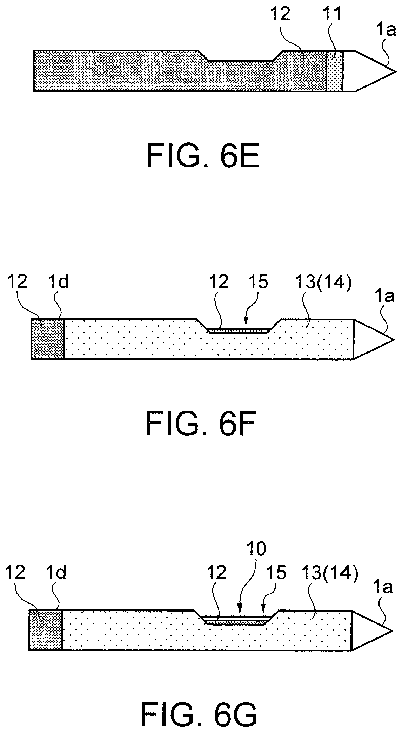

Subsequently, in a wiring step shown in Fig. 6E, the electrode layer 12, which is a conductive layer to be formed as a wire, is formed on the outer surface (the surface section 1b and the inclined surface sections 1c) of the first insulting film 11. Note that the electrode layer 12 functions as a wire that causes the detection film 10 and a control section of the measuring device 50 to electrically conduct. In this embodiment, the electrode layer 12 of platinum is formed by electroless platinum plating. Electrolytic platinum plating may be used.

Subsequently, in a second insulating film forming step shown in Fig. 6F, the second insulating film 13 is formed on the outer surface of the electrode layer 12 excluding a connect section 1d electrically conducted to the needle tip, the surface section 1b, and the measuring device 50. Note that the second insulating film 13 is also formed on the outer surface of the electrode layer 12 of the inclined surface sections 1c. The second insulating film 13 corresponds to the insulating section 14. Consequently, the electrode window section 15 in which the electrode layer 12 is exposed is formed. With this configuration, the insulating section 14 can be considered as including the inclined surface sections 1c inclining toward the electrode window section 15.

Note that synthetic resin same as the synthetic resin of the first insulating film 11 can be used as the insulating material. In this embodiment, Teflon (registered trademark) work is performed to form a film of Teflon (fluororesin) . By using the Teflon in the second insulating film 13, it is possible to provide, in the first electrode needle 1, characteristics such as chemical resistance, a low elution property, non-adhesiveness, electric insulation, and abrasion resistance of the Teflon. In particular, since the Teflon has an extremely low coefficient of friction, it is possible to reduce resistance even when the first electrode needle 1 is punctured into the skin surface 60c and when the first electrode needle 1 is pulled out from the skin surface 60c. Therefore, it is possible to prevent the subject 60 from easily feeling pain.

In this embodiment, the second insulating film 13 (the insulating section 14) is formed in regions corresponding to the inclined surface sections 1c. However, the second insulating film 13 does not have to be formed in the regions corresponding to the inclined surface sections 1c. In this case, the outer circumferential section of the surface section 1b and the regions of the inclined surface sections 1c may be, for example, covered with a partition wall layer 16.

Subsequently, in a sensor forming step shown in Fig. 6G, the detection film 10 is formed on the surface section 1b (the upper surface of the exposed electrode layer 12).

The detection film 10 is explained with reference to Fig. 7. The detection film 10 is configured by the electrode layer 12, the partition wall layer 16, and a sensing layer 17. In the detection film 10, an enzyme electrode layer is set on the upper surface of the electrode layer 12. In this embodiment, the exposed electrode layer 12 functions as the enzyme electrode layer. The partition wall layer 16 is set on the upper surface of the electrode layer 12 and in the region of the electrode layer 12 corresponding to the outer circumferential section of the surface section 1b. The partition wall layer 16 is formed in a state in which the inner side thereof is opened. The electrode layer 12 functioning as the enzyme electrode layer is exposed on the opened inner side.

The sensing layer 17 is set over the opened electrode layer 12 and the partition wall layer 16. The sensing layer 17 is stacked on the electrode layer 12 on the inner side of the partition wall layer 16. The sensing layer 17 is formed by four layers. In the sensing layer 17, a noise removing layer 17A, a sensing layer for sensing information in an organism, a detection layer 17B functioning as an enzyme layer, a protection layer 17C, and an adjustment layer 17D are stacked in this order from the electrode layer 12 side.

The partition wall layer 16 is set in order to improve adhesion of the enzyme electrode layer (the electrode layer 12) and the sensing layer 17. The sensing layer 17 has a hydrophilic nature and absorbs the interstitial fluid to swell. The swelled sensing layer 17 easily peels from the electrode layer 12. The partition wall layer 16 only has to be made of a material from which the swelled sensing layer 17 less easily peels. The material of the partition wall layer 16 is not particularly limited. As the material of the partition wall layer 16, for example, polyimide and acrylic can be used.

The noise removing layer 17A prevents compounds of acetaminophen, ascorbic acid, uric acid, and the like from permeating through the detection film 10 to reach the enzyme electrode layer. The compounds of acetaminophen, ascorbic acid, uric acid, and the like are likely to be included in the interstitial fluid. In order to realize this function, the material of the noise removing layer 17A is not particularly limited. However, methylcellulose, acetylcellulose (cellulose acetate), polyvinylpyrrolidone, polyvinyl alcohol, and the like can be used.

The detection layer 17B is a layer containing an enzyme. The detection layer 17B includes the enzyme, resin including the enzyme, a binder or a hardener, and albumin for protecting and stabilizing the enzyme. The enzyme includes glucose oxidase. The glucose oxidase facilitates the enzyme reaction indicated by Formula (1) above.

The protection layer 17C protects an upper interface of the detection layer 17B. In order to realize the function, the material of the protection layer 17C is not particularly limited. However, methylcellulose, acetylcellulose (cellulose acetate), polyvinylpyrrolidone, polyvinyl alcohol, polyvinyl alcohol-polyvinyl acetate copolymer, and the like can be used alone or in combination. Further, albumin is desirably contained in the material of the protection layer 17C.

The adjustment layer 17D prevents blood, interstitial fluid, and the like, which are measurement targets, from coming into direct contact with the detection layer 17B. The adjustment layer 17D has a function of causing a part of oxygen and glucose to permeate and controlling permeability of the oxygen and the glucose. The adjustment layer 17D desirably causes the oxygen to permeate more than the glucose.

When the glucose exceeding detectable concentration reaches the detection layer 17B, the enzyme reaction changes to a saturated state. In this state, a glucose value cannot be detected. Therefore, the adjustment layer 17D controls the permeability of the glucose and suppresses the enzyme reaction from changing to the saturated state even when measurement target glucose concentration is high.

The material of the adjustment layer 17D only has to be capable of controlling the permeability of the oxygen and the glucose and is not particularly limited. As the material of the adjustment layer 17D, a material formed by generating urethane bond and constructing a bridge structure using a crosslinking agent such as an isocyanate compound and polyethylene glycol of polymer of a terminal hydroxyl group, acrylic acid 4-hydroxybutyl, or the like alone or as a mixture can be used.

The first electrode needle 1 is formed through the steps explained above.

Fig. 8 is a block diagram schematically showing a circuit configuration of the measuring device 50. The measuring device 50 includes a current detection circuit 56 functioning as a current detecting section. The first electrode needle 1, the second electrode needle 2, and the third electrode needle 3 are connected to the current detection circuit 56. The current detection circuit 56 is further connected to an A/D (Analog/Digital) conversion circuit 57. The current detecting circuit 56 detects, according to an instruction of a measurement control section 55, an electric current flowing between the first electrode needle 1 (the detection film 10) and the second electrode needle 2. The current detection circuit 56 converts the electric current into a voltage and outputs the voltage to the A/D conversion circuit 57. The electric current flowing between the detection film 10 and the second electrode needle 2 has a correlation with a glucose value. Therefore, the voltage output to the A/D conversion circuit 57 has a correlation with the glucose value.

The A/D conversion circuit 57 converts the voltage into a numerical value. The A/D conversion circuit 57 outputs data of the voltage converted into the numerical value to the measurement control section 55 having a function of a computing section. The measurement control section 55 converts the input voltage data into a glucose value. The measurement control section 55 has stored therein data of a correlation table indicating a relation between the voltage data and the glucose value. The measurement control section 55 computes the glucose value using the correlation table and the voltage data.

The measurement control section 55 outputs the computed glucose value to the display section 51. The display section 51 displays the glucose value. Further, the measurement control section 55 has stored therein data of a determination value. The measurement control section 55 compares the determination value and the glucose value and determines whether the glucose value is a value within a normal range or in an abnormal state. When determining that the glucose value is an abnormal value, the measurement control section 55 outputs information indicating the abnormal state to the display section 51 and the speaker 53. The display section 51 displays a warning sentence or the like. The speaker 53 emits warning sound.

The operator operates the input sections 52 to input an instruction for a measurement start, a measurement stop, or the like to the measurement control section 55. The measurement control section 55 drives the measuring device 50 according to the instruction of the operator. A battery 58 is set in the measuring device 50. The battery 58 supplies electric power to the other components including the measurement control section 55. Consequently, the measuring device 50 can be set in the subject 60 and driven without being connected to an external power supply. Therefore, the subject 60 can easily move in a state in which the subject 60 wears and operates the measuring device 50.

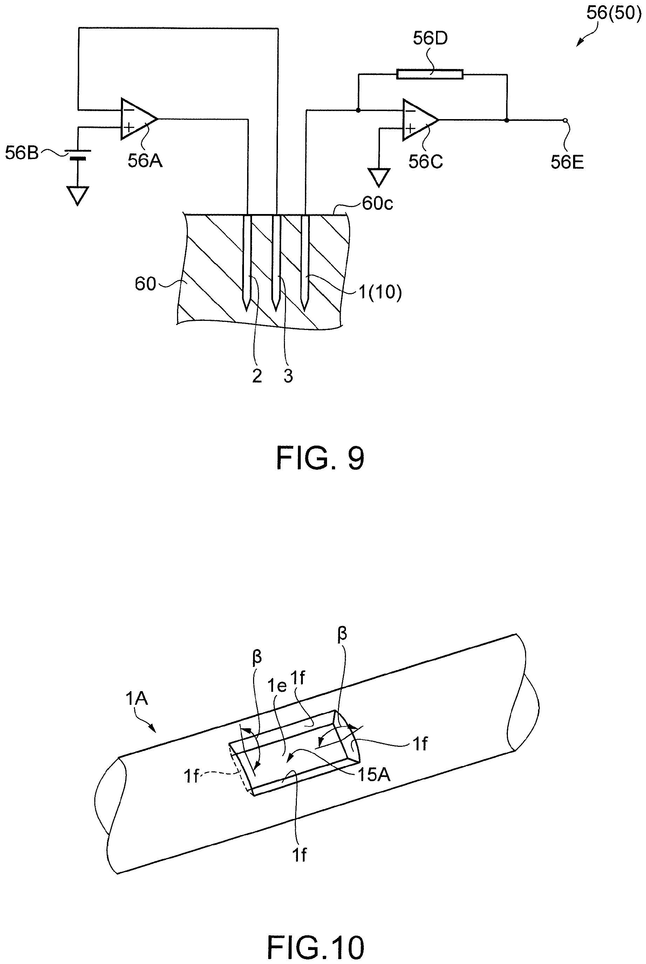

Fig. 9 is a circuit diagram showing the configuration of the current detection circuit 56.

The current detection circuit 56 shown in Fig. 9 is a circuit called potentiostat. The current detection circuit 56 includes a first operational amplifier 56A. A power supply 56B is connected to a plus input end of the first operational amplifier 56A. A predetermined voltage is applied to the plus input end of the first operational amplifier 56A. A voltage value is not particularly limited. However, in this embodiment, the voltage value is set to, for example, 0.6 V. The power supply 56B can be realized by dividing the voltage of the battery 58 and setting a voltage in a diode. The third electrode needle 3 is connected to a minus input end of the first operational amplifier 56A. The second electrode needle 2 is connected to an output end of the first operational amplifier 56A. The first operational amplifier 56A feeds an electric current the second electrode needle 2 such that potential from the third electrode needle 3 and potential given by the power supply 56B become the same potential. The first operational amplifier 56A acts such that an electric current does not flow to the third electrode needle 3.

The current detection circuit 56 includes a second operational amplifier 56C. A plus input end of the second operational amplifier 56C is grounded. The detection film 10 is connected to a minus input end of the second operational amplifier 56C. The minus input end and an output end of the second operational amplifier 56C are connected via a resistor 56D. An output terminal 56E of the current detection circuit 56 is connected to the output end of the second operational amplifier 56C.

In the second operational amplifier 56C, the plus input end and the minus input end have the same potential. Therefore, the potential of the detection film 10 is 0 V. Potential of 0.6 V is generated between the third electrode needle 3 and the detection film 10. In the subject 60, the enzyme reaction indicated by Formula (1) to Formula (3) occurs. Therefore, an electric current flows between the second electrode needle 2 and the detection film 10. Since the second operational amplifier 56C has high input impedance, the electric current flows from the detection film 10 to the output end of the second operational amplifier 56C passing through the resistor 56D. Since a voltage drop occurs when the electric current flows to the resistor 56D, in the output terminal 56E, the electric current between the second electrode needle 2 and the detection film 10 is converted into a voltage and output.

A not-shown compensation circuit is set in the current detection circuit 56. The compensation circuit detects resistance between the third electrode needle 3 and the detection film 10. The resistance increases when films are formed on the surfaces of the second electrode needle 2, the third electrode needle 3, and the detection film 10. The compensation circuit changes, according to the resistance between the third electrode needle 3 and the detection film 10, a voltage applied to the second electrode needle 2. Therefore, stable current measurement can be performed by the compensation circuit.

According to the embodiment explained above, effects explained below can be obtained.
(1) The first electrode needle 1 in this embodiment includes the core section 1a, the electrode (the electrode layer 12) formed in the core section 1a, the insulating section 14 in which the electrode layer 12 is covered with the second insulating film 13 formed of the insulating material, and the electrode window section 15 in which the electrode layer 12 or the sensing layer 17 including the sensing layer (the detection layer 17B) is exposed. Consequently, since the region other than the electrode window section 15 is covered with the insulting section 14, when the first electrode needle 1 is punctured into an organism and used, it is possible to reduce a loss of a voltage, noise, and the like and accurately detect an electric current. Consequently, it is possible to realize an electrode needle that can highly accurately measure information in the organism. Similarly, the second electrode needle 2 includes the core section 2a, the electrode layer 22, the insulting section 24, and the electrode window section 25 in which the electrode layer 22 is exposed. Therefore, the second electrode needle 2 achieves the same effects. The third electrode needle 3 achieves effects same as the effects of the second electrode needle 2.
(2) In the first electrode needle 1 in this embodiment, the electrode window section 15 is exposed in the rectangular shape along the puncturing direction in which the first electrode needle 1 is punctured. Therefore, it is possible to prevent peeling of, for example, the electrode layer 12 and the sensing layer 17 including the sensing layer (the detection layer 17B) during the puncture. Since the surface section 1b forming the base of the electrode window section 15 is formed in the rectangular shape, it is easy to form the electrode layer 12 and the sensing layer.
(3) In the first electrode needle 1 in this embodiment, since the core section 1a or the insulating section 14 includes the inclined surface sections 1c inclining toward the electrode window section 15, it is possible to reduce resistance in inserting and pulling out the first electrode needle 1. Therefore, it is possible to realize an electrode needle that prevents a patient from easily feeling pain.
   In the first electrode needle 1 in this embodiment, since the detection layer 17B functioning as the sensing layer is provided, the detection layer 17B can sense glucose serving as biological information and feed an electric current.
(5) In the first electrode needle 1 in this embodiment, the major diameter D of the first electrode needle 1 is 0.1 mm or more. Note that the major diameter D indicates a diameter in a place where the diameter is the largest in a sectional shape of the electrode needle. In this case, since the first electrode needle 1 sticks into the skin without bending, it is possible to prevent the electrode needle from being easily broken. The major diameter D is 0.5 mm or less. In this case, since a stimulus of the electrode needle to a pain spot is small, it is possible to prevent a patient from easily feeling pain. Therefore, it is possible to realize the first electrode needle 1 that is less easily broken and prevents the patient from easily feeling pain.
(6) In the first electrode needle 1 in this embodiment, since the angles formed by the electrode window section 15 and the inclined surface sections 1c is 90° or more and 170° or less, it is possible to reduce resistance in inserting and pulling out the first electrode needle 1. It is possible to smoothly perform the insertion and the pull-out.
(7) The measuring device 50 functioning as the biological information measuring device in this embodiment includes the first electrode needle 1, the second electrode needle 2, and the third electrode needle 3. It is possible to measure a glucose value by detecting, with the current detection circuit 56, an electric current between the first electrode needle 1 and the second electrode needle 2. When a substance included in the interstitial fluid adheres to the surfaces of the first electrode needle 1 and the second electrode needle 2 and films are formed, it is possible to improve measurement accuracy of a current value in the current detection circuit 56 by detecting resistance with the third electrode needle 3. Compared with when the needle including the substrate on which the first electrode, the second electrode, and the like are set is used as in the configuration in the past, it is possible to reduce the major diameter D of the first electrode needle 1, the second electrode needle 2, and the third electrode needle 3 inserted into the skin. Note that, as the major diameter D is smaller, a pain spot of the skin is less easily stimulated. Therefore, in the measuring device 50, even if the first electrode needle 1, the second electrode needle 2, and the third electrode needle 3 are inserted into or pulled out from the abdomen or the like of a patient in order to measure a glucose value in the organism, it is possible to prevent the patient from easily feeling pain.
(8) With the measuring device 50 in this embodiment, since the first electrode needle 1 is the working electrode including the enzyme layer (the detection layer 17B) as the sensing layer, glucose reacts with the enzyme layer to generate an electric current. The second electrode needle 2 is the counter electrode that leads the electric current generated in the first electrode needle 1. Note that, as glucose concentration is higher, the current value is larger because the reaction with the detection layer 17B further advances. Therefore, it is possible to accurately detect the concentration of the glucose by analyzing the current value detected by the current detection circuit 56. Further, the measuring device 50 includes the third electrode needle 3 functioning as the reference electrode used for detection of the resistance of the electric current flowing to the first electrode needle 1. Therefore, it is possible to improve measurement accuracy of the current value in the current detection circuit 56.

### Second Embodiment

Fig. 10 is a general perspective view schematically showing another example of a surface section according to a second embodiment.

A surface section 1e of a first electrode needle 1A in this embodiment is different from the surface section 1b in the first electrode needle 1 in the first embodiment as explained below. On the surface section 1b in the first embodiment, the inclined surface sections 1c extending along the longitudinal direction (the puncturing direction) of the core section 1a are formed. Inclined surface sections extending along the latitudinal direction are not formed. On the other hand, on the surface section 1e in this embodiment, inclines surface sections 1f are formed along the longitudinal direction and, in addition, the inclined surface sections 1f are formed along the latitudinal direction as well.

Sections other than the surface section 1e are configured the same as the sections in the first embodiment. Therefore, on the surface section 1e including the inclined surface sections 1f, an electrode window section 15A is formed like the electrode window section 15 in the first embodiment. Note that angles β formed by the surface section 1e (the electrode window section 15A) and the inclined surface sections 1f are set to 90° or more and 170° or less as in the first embodiment. In this embodiment, specifically, the angles P are set to 135°.

In the first electrode needle 1A having such a configuration and a measuring device including the first electrode needle 1A, it is possible to achieve effects same as the effects (1) to (8) in the first embodiment.

### Third Embodiment

Another measuring device 70 functioning as a biological information measuring device is explained.

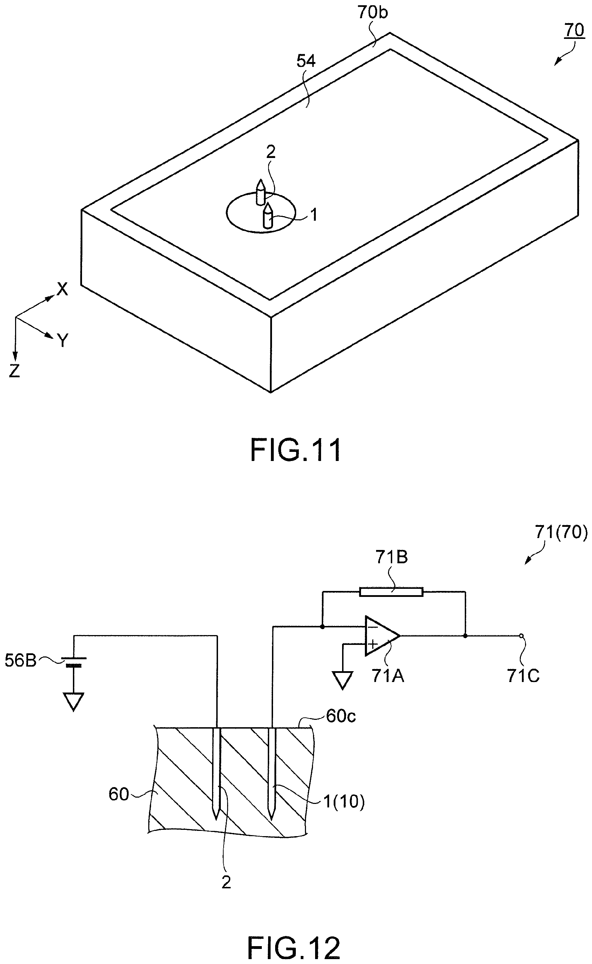

Fig. 11 is a schematic perspective view of the measuring device 70 according to a third embodiment. Fig. 12 is a circuit diagram showing the configuration of a current detection circuit 71.

This embodiment is different from the first embodiment in that the third electrode needle 3, which is the reference electrode, is omitted. Note that explanation is omitted concerning components same as the components in the first embodiment.

As shown in Fig. 11, in the measuring device 70 in this embodiment, the first electrode needle 1 and the second electrode needle 2 are set on a rear surface 70b. The first electrode needle 1 and the second electrode needle 2 are stuck into the skin of the subject 60 and used. Note that the first electrode needle 1 and the second electrode needle 2 in this embodiment are configured the same as those in the first embodiment. Similarly, the first electrode needle 1 functions as the working electrode and the second electrode needle 2 functions as the counter electrode.

As shown in Fig. 12, the measuring device 70 includes the current detection circuit 71 functioning as the current detecting section. The current detection circuit 71 is a circuit that measures an electric current flowing between the second electrode needle 2 and the detection film 10. The current detection circuit 71 includes an operational amplifier 71A. A plus input end of the operational amplifier 71A is grounded. The detection film 10 is connected to a minus input end of the operational amplifier 71A. The minus input end and an output end of the operational amplifier 71A are connected via a resistor 71B. An output terminal 71C of the current detection circuit 71 is connected to the output end of the operational amplifier 71A.

The second electrode needle 2 is connected to the power supply 56B. In the subject 60, the enzyme reaction indicated by Formula (1) to Formula (3) occurs. Therefore, an electric current flows between the second electrode needle 2 and the detection film 10. The electric current flowing between the second electrode needle 2 and the detection film 10 is input to the output end of the operational amplifier 71A passing through the resistor 71B. The electric current input to the output end flows from the ground to the second electrode needle 2. The voltage at the minus input terminal of the operational amplifier 71A is 0 V. The voltage at the output terminal 71C is, according to a voltage drop of the resistor 71B, a value obtained by multiplying together a resistance value of the resistor 71B and a value of the electric current flowing in the resistor 71B.

Since the input end of the operational amplifier 71A has high impedance, the electric current flowing between the second electrode needle 2 and the detection film 10 is equal to the electric current flowing in the resistor 71B. Therefore, by measuring the voltage at the output terminal 71C, it is possible to measure the electric current flowing between the second electrode needle 2 and the detection film 10. The second electrode needle 2 is equivalent to the counter electrode and the detection film 10 is equivalent to the working electrode. Therefore, by measuring the voltage at the output terminal 71C, it is possible to measure a glucose value of the subject 60.

As explained above, according to this embodiment, effects explained below can be obtained.

The measuring device 70 in this embodiment includes the first electrode needle 1 and the second electrode needle 2. It is possible to measure biological information (a glucose value) by detecting, with the current detection circuit 71, an electric current between the first electrode needle 1 and the second electrode needle 2. Since the first electrode needle 1 and the second electrode needle 2 are configured as in the first embodiment, even if the first electrode needle 1 and the second electrode needle 2 are punctured, it is possible to measure the glucose value without stimulating the pain spot of the subject 60. Therefore, the measuring device 70 in this embodiment can measure the biological information (the glucose value) while causing a patient to less easily feel pain even if the first electrode needle 1 and the second electrode needle 2 are punctured.

### Fourth Embodiment

An insulin supply device 80 functioning as a liquid supply device is explained. In the insulin supply device 80 in a fourth embodiment, the measuring device 50 explained in the first embodiment is used. Note that explanation is omitted concerning components same as the components in the first embodiment.

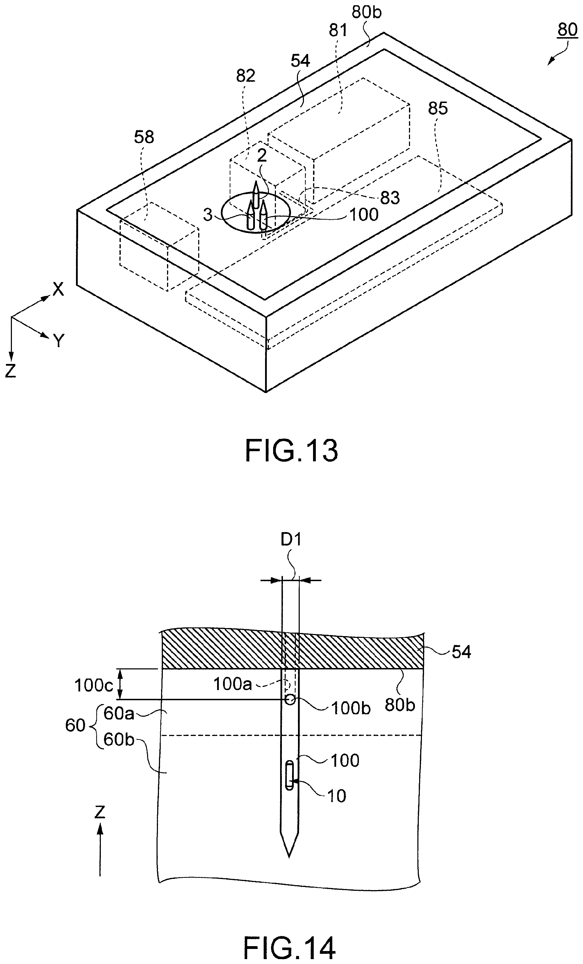

Fig. 13 is a general perspective view schematically showing the structure of the insulin supply device 80 according to the fourth embodiment. Specifically, Fig. 13 is a diagram of the insulin supply device 80 viewed from the rear side.

In this embodiment, as shown in Fig. 13, on a rear surface 80b of the insulin supply device 80, a first electrode needle 100 functioning as an injection needle and a working electrode, the second electrode needle 2, and the third electrode needle 3 are set.

On the inside of the insulin supply device 80, a tank 81 that stores insulin serving as liquid is set. Further, on the inside of the insulin supply device 80, a pump 82 that feeds the insulin stored in the tank 81 to the first electrode needle 100 is set. The pump 82 and the first electrode needle 100 are coupled by a tube 83 for transportation. Besides, on the inside of the insulin supply device 80, the battery 58, a circuit board 85, and the like are set.

The tank 81 includes a bag made of resin on the inside. The insulin is stored in the inside of the bag. The bag is deformed when the insulin in the tank 81 decreases. Consequently, it is possible to store the insulin while preventing the insulin from coming into contact with the air. The pump 82 is desirably a volume pump that can manage a transportation amount. A gear pump, a screw pump, a vane pump, and the like can be used. On the circuit board 85, a circuit that controls the pump 82 and a circuit that measures a glucose value as in the first embodiment are set.

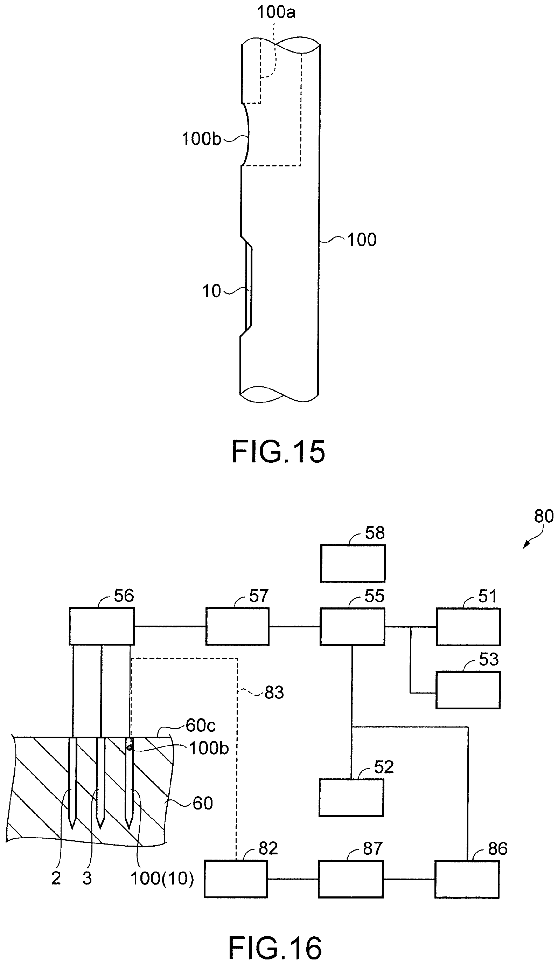

Fig. 14 is a general side view schematically showing the structure of the first electrode needle 100. Fig. 15 is a main part side view schematically showing the structure of the first electrode needle 100.

As shown in Figs. 14 and 15, the first electrode needle 100 is formed the same as the first electrode needle 1 in the first embodiment. A major diameter D1 of the first electrode needle 100 is 0.1 mm or more and 0.5 mm or less. Therefore, the electrode needle 100 of the insulin supply device 80 is less easily broken and can prevent the subject 60 from easily feeling pain.

In the first electrode needle 100, the detection film 10 is set as in the first embodiment. The first electrode needle 100 is different from the first embodiment in that, on the inside of the first electrode needle 100, a hole section 100a is set as a channel solid at a distal end formed from the rear end face of the first electrode needle 100 toward the distal end portion along the center axis of the first electrode needle 100. The hole section 100a is connected to an opening section 100b functioning as a lateral hole formed to be opened in a part between the rear end face of the first electrode needle 100 and the detection film 10.

When the pump 82 is driven, the insulin flows out from the tank 81, circulates through the pump 82, the tube 83, and the hole section 100a functioning as the channel of the first electrode needle 100 in this order, and is discharged to the subject 60 from the opening section 100b of the first electrode needle 100. A distance 100c between the center of the opening section 100b and the rear surface 80b is set in a range of 1 mm or more and 2 mm or less. In this case, since the opening section 100b is located in the skin tissue 60a, it is possible to supply the insulin to the skin tissue 60a. Since the detection film 10 is located in the subcutaneous tissue 60b, it is possible to measure a glucose value in the subcutaneous tissue 60b.

The interstitial fluid is present more in the subcutaneous tissue 60b compared with the skin tissue 60a. The detection film 10 more often comes into contact with the glucose when the detection film 10 is located in a place where the interstitial fluid is present more. Therefore, compared with when the detection film 10 is located in the skin tissue 60a, it is possible to more accurately measure a glucose value when the detection film 10 is located in the subcutaneous tissue 60b compared with when the detection film 10 is located in the skin tissue 60a.

Fig. 16 is a block diagram schematically showing a circuit configuration of the insulin supply device 80.

On the circuit board 85, as a biological-information measuring section, as in the first embodiment, the measuring control section 55 having the function of the computing section, the current detection circuit 56, the A/D conversion circuit 57, the display section 51, the input sections 52, and the speaker 53 are provided as in the first embodiment. Besides, on the circuit board 85, a supply-amount control section 86 that controls a supply amount of the insulin and a pump driving section 87 that drives the pump 82 are provided. The supply-amount control section 86 is connected to the measurement control section 55 and further connected to the pump driving section 87. The pump driving section 87 is connected to the pump 82.

The first electrode needle 100, the second electrode needle 2, and the third electrode needle 3 piece through parts respectively corresponding thereto on the skin surface 60c to be inserted (punctured) into the subject 60.

The measurement control section 55 computes the measured glucose value and compares the glucose value with the determination value. When determining that the glucose value is in an abnormal state, the measurement control section 55 outputs an instruction signal for discharging the insulin and data of the glucose value to the supply-amount control section 86. The supply-amount control section 86 computes an amount of the insulin supplied to the subject 60. The supply-amount control section 86 includes a supply amount table indicating a relation between the glucose value and the amount of the insulin to be supplied. The supply-amount control section 86 computes the amount of the insulin to be applied with reference to the glucose value and the supply amount table.

Further, the supply-amount control section 86 computes, from the amount of the insulin to be supplied, time in which the pump 82 is driven. An amount of the insulin to be supplied per unit time is set in the pump 82. The supply-amount control section 86 outputs, to the pump driving section 87, data of the time in which the pump 82 is driven and an instruction signal for driving the pump 82. The pump driving section 87 drives the pump 82 according to the instruction signal. The pump 82 supplies a designated amount of the insulin to the subject 60.

According to the embodiment explained above, effects explained below can be obtained.

In the first electrode needle 100 in this embodiment, the hole section 100a functioning as the channel for circulating the liquid (the insulin) to the core section and the opening section 100b connected to the hole section 100a are set. Consequently, it is possible to not only measure an amount of the glucose in the organism but also include a function of the injection needle that supplies the insulin into the organism.

The insulin supply device 80 functioning as the liquid supply device in this embodiment includes the biological-information measuring device (the measuring device 50) in the first embodiment, the first electrode needle 100 including the hole section 100a functioning as the channel, the second electrode needle 2, and the third electrode needle 3. Consequently, it is possible to realize the insulin supply device 80 that prevents a patient from easily feeling pain. Besides, it is possible to achieve simplification of the insulin supply device 80. It is possible to improve convenience.

### Fifth Embodiment

An insulin supply device 90, which is another example of the liquid supply device, is explained.

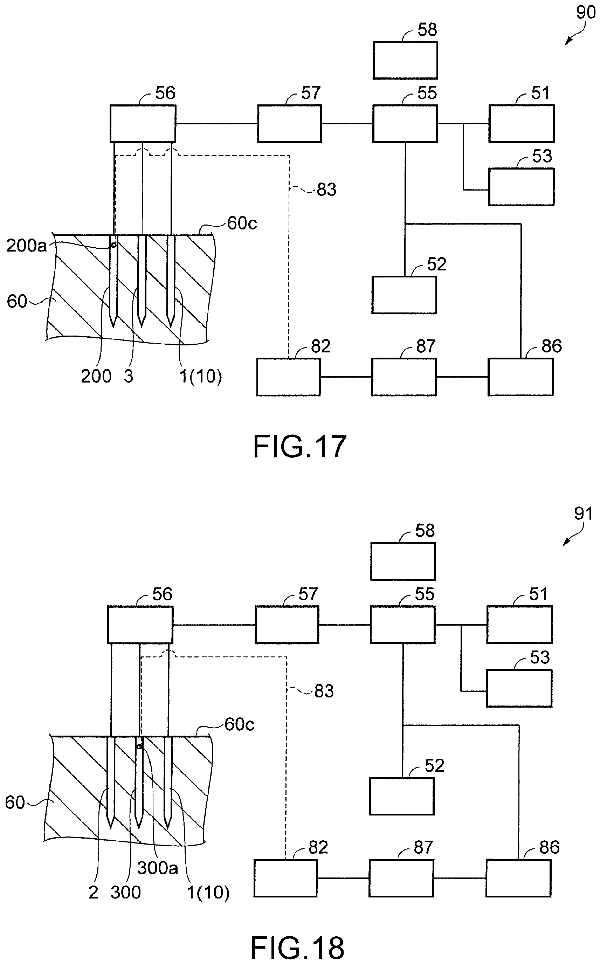

Fig. 17 is a block diagram schematically showing a circuit configuration of the insulin supply device 90 according to a fifth embodiment.

The insulin supply device 90 in this embodiment is different from the insulin supply device 80 in the fourth embodiment as explained below. In the insulin supply device 80 in the fourth embodiment, the hole section 100a functioning as the channel and the opening section 100b are formed in the first electrode needle 100. On the other hand, in the insulin supply device 90 in this embodiment, a hole section functioning as a channel and an opening section 200a are formed in a second electrode needle (a second electrode needle 200) functioning as a counter electrode.

Specifically, in this embodiment, as shown in Fig. 17, the insulin supply device 90 functioning as the liquid supply device includes the second electrode needle (the second electrode needle 200) functioning as the counter electrode. A hole section functioning as a channel is set on the inside of the second electrode needle 200. The hole section is connected to the opening section 200a set on the side surface of the second electrode needle 200. The tube 83 is connected to the hole section of the second electrode needle 200. Other circuit components are the same as those in the fourth embodiment.

When the pump 82 is driven, the insulin flows out from the tank 81 and is supplied from the opening section 200a of the second electrode needle 200 to the subject 60 passing through the pump 82, the tube 83, and the hole section inside the second electrode needle 200.

When the detection film 10 reacts with the glucose, an electric current flows between the detection film 10 and the second electrode needle 200. The current detection circuit 56 can detect a glucose value in the interstitial fluid by detecting an electric current. When the measurement control section 55 determines that the glucose value in the subject 60 is in an abnormal state, the supply-amount control section 86 supplies the insulin from the opening section 200a of the second electrode needle 200 to the subject 60.

According to the embodiment explained above, effects explained below can be obtained.

In the second electrode needle 200 in this embodiment, the hole section (the opening section 200a) functioning as the channel for circulating the liquid (the insulin) is set in the core section. Consequently, it is possible to not only measure an amount of glucose in an organism but also include a function of an injection needle that supplies the insulin into the organism.

The insulin supply device 90 functioning as the liquid supply device in this embodiment includes the biological information measuring device (the measuring device 50) in the first embodiment, the first electrode needle 1 functioning as the working electrode, the second electrode needle 200 functioning as the counter electrode including the hole section functioning as the channel and the opening section 200a, and the third electrode needle 3 functioning as the reference electrode. Consequently, the insulin supply device 90 does not need to separately include an injection needle. Therefore, it is possible to realize the insulin supply device 90 that prevents a patient from easily feeling pain. Besides, it is possible to achieve simplification of the insulin supply device 90. It is possible to improve convenience.

### Sixth Embodiment

An insulin supply device 91, which is another example of the liquid supply device, is explained.

Fig. 18 is a block diagram schematically showing a circuit configuration of the insulin supply device 91 according to a sixth embodiment.

The insulin supply device 91 in this embodiment is different from the insulin supply device 90 in the fifth embodiment as explained below. In the insulin supply device 90 in the fifth embodiment, the hole section functioning as the channel and the opening section 200a are formed in the second electrode needle 200. On the other hand, in the insulin supply device 91 in this embodiment, a hole section functioning as a channel and an opening section 300a are formed in a third electrode needle (a third electrode needle 300) functioning as a reference electrode.

Specifically, in this embodiment, as shown in Fig. 18, the insulin supply device 91 functioning as the liquid supply device includes the third electrode needle (the third electrode needle 300) functioning as the reference electrode. A hole section functioning as a channel is set on the inside of the third electrode needle 300. The hole section is connected to the opening section 300a set on the side surface of the third electrode needle 300. The tube 83 is connected to the hole section of the third electrode needle 300. Other circuit components are the same as those in the fifth embodiment.

When the pump 82 is driven, the insulin flows out from the tank 81 and is supplied from the opening section 300a of the third electrode needle 300 to the subject 60 passing through the pump 82, the tube 83, and the hole section on the inside of the third electrode needle 300.

When the detection film 10 reacts with the glucose, an electric current flows between the detection film 10 and the second electrode needle 2. The current detection circuit 56 can detect a glucose value in the interstitial fluid by detecting an electric current. When the measurement control section 55 determines that the glucose value in the subject 60 is in an abnormal state, the supply-amount control section 86 supplies the insulin from the opening section 300a of the third electrode needle 300 to the subject 60.

According to the embodiment explained above, effects explained below can be obtained.

In the third electrode needle 300 in this embodiment, the hole section (the opening section 300a) functioning as the channel for circulating the liquid (the insulin) is set in the core section. Consequently, it is possible to not only measure an amount of glucose in an organism but also include a function of an injection needle that supplies the insulin into the organism.

The insulin supply device 91 functioning as the liquid supply device in this embodiment includes the biological information measuring device (the measuring device 50) in the first embodiment, the first electrode needle 1 functioning as the working electrode, the second electrode needle 2 functioning as the counter electrode, and the third electrode needle 300 functioning as the reference electrode including the hole section functioning as the channel and the opening section 300a. Consequently, the insulin supply device 91 does not need to separately include an injection needle. Therefore, it is possible to realize the insulin supply device 91 that prevents a patient from easily feeling pain. Besides, it is possible to achieve simplification of the insulin supply device 91. It is possible to improve convenience.

### Seventh Embodiment

An insulin supply device 92, which is another example of the liquid supply device, is explained.

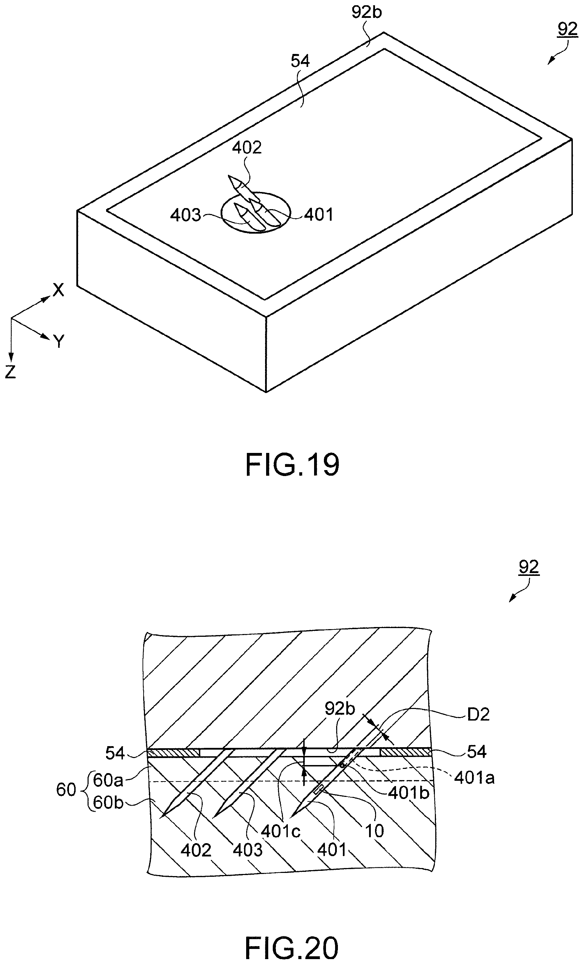

Fig. 19 is a schematic perspective view showing the structure of the insulin supply device 92 according to a seventh embodiment and is a view of the insulin supply device 92 functioning as the liquid supply device viewed from the rear side. Fig. 20 is a side sectional view schematically showing the structure of a first electrode needle 401 to a third electrode needle 403 and is a view of the first electrode needle 401 to the third electrode needle 403 punctured into the subject 60.

The insulin supply device 92 in this embodiment is different from the insulin supply device 80 in the fourth embodiment in that the first electrode needle 401 to the third electrode needle 403 are punctured obliquely to the surface of a skin. Note that explanation is omitted concerning components same as the components in the fourth embodiment.

In this embodiment, as shown in Fig. 19, on a rear surface 92b of the insulin supply device 92, a first electrode needle (the first electrode needle 401) functioning as an injection needle and a working electrode, a second electrode needle (the second electrode needle 402) functioning as a counter electrode, and a third electrode needle (the third electrode needle 403) functioning as a reference electrode are set. The first electrode needle 401, the second electrode needle 402, and the third electrode needle 403 are set obliquely to a rear surface 92b and set in parallel to one another.

In Fig. 20, a major diameter D2 of the first electrode needle 401 is 0.1 mm or more and 0.5 mm or less. Therefore, the first electrode needle 401 of the insulin supply device 92 is less easily broken and can prevent the subject 60 from easily feeling pain. In the first electrode needle 401, the detection film 10 is set from the center toward the distal end side. A hole section 401a is set on the inside of the first electrode needle 401. The hole section 401a is connected to an opening section 401b functioning as a hole section located between the base of the first electrode needle 401 and the detection film 10.

When the pump 82 is driven, the insulin flows out from the tank 81 and flows out from the opening section 401b of the first electrode needle 401 to the subject 60 passing through the pump 82, the tube 83, and the hole section 401a functioning as the channel. A distance 401c between the center of the opening section 401b and the rear surface 92b is set in a range of 1 mm or more and 2 mm or less. In this case, since the opening section 401b is located in the skin tissue 60a, it is possible to supply the insulin to the skin tissue 60a. It is possible to measure a glucose value in the subcutaneous tissue 60b. The interstitial fluid is present more in the subcutaneous tissue 60b than in the skin tissue 60a. The detection film 10 more often comes into contact with the glucose in a place where the interstitial fluid is present more. Therefore, it is possible to accurately measure the glucose value.

A major diameter of the second electrode needle 402 and the third electrode needle 403 is 0.1 mm or more and 0.5 mm or less. Therefore, the second electrode needle 402 and the third electrode needle 403 of the insulin supply device 92 are less easily broken and can prevent the subject 60 from easily feeling pain. The first electrode needle 401, the second electrode needle 402, and the third electrode needle 403 are obliquely inserted into the subject 60. Consequently, when force in the normal direction of the rear surface 92b acts on the insulin supply device 92, the direction of the force and the longitudinal direction of the needles cross. Therefore, the skin tissue 60a acts such that the first electrode needle 401, the second electrode needle 402, and the third electrode needle 403 do not come off. As a result, even when external force is applied to the insulin supply device 92, it is possible to prevent the insulin supply device 92 from being easily separated from the subject 60.

Note that the invention is not limited to the embodiments explained above. The invention can be carried out with various changes, improvements, and the like applied to the embodiments without departing from the spirit of the invention. Modifications are explained below.

In the first embodiment, in the first electrode needle 1, a part of the core section 1a includes the surface section 1b to form the electrode window 15. In the second electrode needle 2, the region 2b having the predetermined width W is exposed around the core section 2a to form the electrode window section 25. However, a surface section same as the surface section 1b may be provided in the core section 2a of the second electrode needle 2 to form the electrode window section 25. This is the same in the third electrode needle 3.

In the insulin supply device 80 in the fourth embodiment, the three electrode needles are used. However, only the first electrode needle 100 functioning as the working electrode and the second electrode needle 2 functioning as the counter electrode may be used without using the third electrode needle 3 functioning as the reference electrode. In the insulin supply device 90 in the fifth embodiment, only the first electrode needle 1 and the second electrode needle 200 may be used. In the insulin supply device 92 in the seventh embodiment, only the first electrode needle 401 and the second electrode needle 402 may be used. With the insulin supply devices (the liquid supply devices), it is possible to detect an electric current and measure information in an organism. Besides, the insulin supply devices do not need to separately include an injection needle. It is possible to discharge liquid from the channel of the first electrode needle or the second electrode needle. Therefore, it is possible to realize the liquid supply device that prevents a patient from easily feeling pain. Further, it is possible to achieve simplification of the liquid supply device. It is possible to improve convenience.

In the fourth embodiment, the first electrode needle 100 functions as the injection needle. In the fifth embodiment, the second electrode needle 200 functions as the injection needle. In the sixth embodiment, the third electrode needle 300 functions as the injection needle. However, the injection needle is not limited to this. At least two or three of the first electrode needle, the second electrode needle, and the third electrode needle may function as injection needles. In this case, the electrode needles configured as the injection needles may be connected to the tube 83. In this case, the insulin circulating in the tube 83 according to the driving of the pump 82 can circulate through a channel of the electrode needles functioning as the injection needles to be discharged from the opening section. Such a configuration effectively functions, for example, when an amount of the insulin discharged from the injection needles is smaller than an amount necessary in unit time. The configuration is particularly effective when needles have a small major diameter (the needles are thin electrode needles) and a discharge amount per one needle is limited. Note that, by adopting such a configuration, it is possible to expand an adjustment range of a supply amount of the insulin while achieving a reduction in the diameter of the electrode needles in the insulin supply device.

The electrode needles (the first electrode needle 1, the second electrode needle 2, and the third electrode needle 3) in the first embodiment basically include the main bodies circular in section. However, the shape of the main bodies is not limited to this. The sectional shape of the main bodies may be an elliptical shape. This is the same in the second and subsequent embodiments.

The surface section 1b in the first electrode needle 1 in the first embodiment is the surface section substantially rectangular in plan view. However, the surface section 1b is not limited to this. The surface section 1b may be a surface section having a track shape (a shape in which any opposed sides of a rectangle are curved lines and linked to the remaining opposed sides). In this case, the inclined surface sections may be provided between the surface section (the electrode window section) and the core section.

The core sections of the first electrode needle 1, the second electrode needle 2, and the third electrode needle 3 in the first embodiment are formed in the columnar shape. However, the core sections are not limited to this. The core sections may be formed in a tubular shape. When the electrode needles are used as the liquid supply device, it is possible to cause the electrode needles to function as the injection needles by using the portions of tubes as channels in which the liquid circulates.

## Claims

1. An electrode needle adapted to be punctured into an organism, the electrode needle comprising:
a columnar or tubular core section;
an electrode formed in the core section;
an insulating section in which the electrode is covered with an insulating material; and
an electrode window section in which the electrode or a sensing layer formed on the electrode is exposed.

2. The electrode needle according to claim 1, wherein the electrode window section is exposed in a rectangular shape along a puncturing direction in which the electrode needle is punctured.

3. The electrode needle according to claim 1 or 2, wherein the core section or the insulating section includes inclined surface sections inclining toward the electrode window section.

4. The electrode needle according to any one of the preceding claims, wherein the sensing layer is adapted to sense information in the organism.

5. The electrode needle according to any one of the preceding claims, wherein a major diameter of the electrode needle is 0.1 mm or more and 0.5 mm or less.

6. The electrode needle according to any one of claims 3 to 5, wherein angles formed by the electrode window section and the inclined surface sections are 90° or more and 170° or less.

7. The electrode needle according to any one of the preceding claims, wherein the electrode needle includes a channel for circulating liquid.

8. A biological information measuring device comprising:
a first electrode needle and a second electrode needle configured by the electrode needle according to any one of the preceding claims; and
a current detecting section configured to detect an electric current between the first electrode needle and the second electrode needle.

9. The biological information measuring device according to claim 8, wherein
the information in the organism is information concerning glucose,
the first electrode needle is a working electrode including a sensing layer including an enzyme layer, and
the second electrode is a counter electrode that leads an electric current between the second electrode needle and the first electrode needle.

10. The biological information measuring device according to claim 8 or 9, further comprising a third electrode needle functioning as a reference electrode used for detection of resistance of an electric current flowing to the first electrode needle.

11. A liquid supply device comprising the biological information measuring device according to claim 9, wherein
the first electrode needle or the second electrode needle includes a channel for circulating liquid.

12. A liquid supply device comprising the biological information measuring device according to claim 10, wherein
any one of the first electrode needle, the second electrode needle, and the third electrode needle includes a channel for circulating liquid.

13. The liquid supply device according to claim 11, wherein
the information in the organism is information concerning glucose, and
the liquid is insulin.
